Europäisches Patentamt

European Patent Office

Office européen des brevets

(11) Numéro de publication: **0 493 222 B1**

## (12) FASCICULE DE BREVET EUROPEEN

(45) Date de publication du fascicule du brevet: **29.11.95**  (51) Int. Cl.⁶: **A61N 1/365**

(21) Numéro de dépôt: **91403483.0**

(22) Date de dépôt: **20.12.91**

(54) **Procédé de commande d'un stimulateur cardiaque à fréquence de stimulation asservie.**

(30) Priorité: **27.12.90 FR 9016293**

(43) Date de publication de la demande:
**01.07.92 Bulletin 92/27**

(45) Mention de la délivrance du brevet:
**29.11.95 Bulletin 95/48**

(84) Etats contractants désignés:
**BE CH DE ES FR GB IT LI SE**

(56) Documents cités:
EP-A- 0 140 472      EP-A- 0 178 528
EP-A- 0 256 617      EP-A- 0 361 517
WO-A-90/08569       US-A- 4 702 253

(73) Titulaire: **ELA MEDICAL (Société anonyme)**
**98, Rue Maurice Arnoux**
**F-92541 Montrouge (FR)**

(72) Inventeur: **Bonnet, Jean-Luc**
**3, Avenue Richerand**
**F-75010 Paris (FR)**
Inventeur: **Malherbe, Odile**
**18, Rue Galliéni**
**F-94230 Cachan (FR)**

(74) Mandataire: **Laget, Jean-Loup**
**Cabinet Pierre Loyer**
**77, rue Boissière**
**F-75116 Paris (FR)**

Rank Xerox (UK) Business Services
(3.10/3.09/3.3.3)

**Description**

La présente invention concerne un stimulateur cardiaque à fréquence de stimulation asservie.

Cet appareil doit stimuler le coeur du patient en étant capable de modifier la fréquence de stimulation, en fonction de l'activité dudit patient.

On comprend que, si la fréquence de stimulation était constante, le patient ne pourrait pas réagir de façon optimale durant les périodes de repos et les périodes d'effort.

Afin de modifier la fréquence de stimulation, il est usuel de prendre en compte une donnée physiologique du patient. A cet effet, on peut choisir l'une des nombreuses données physiologiques permettant de connaître l'activité physique du patient.

Il est connu par le document EP 0 151 689 de prendre comme donnée physiologique représentative de l'activité du patient la ventilation-minute et de définir la fréquence d'asservissement cardiaque comme une fonction linéaire de la ventilation-minute. La droite représentative de cette fonction présente une pente qui est programmée par le médecin en fonction du patient. Ainsi, la fréquence d'asservissement cardiaque varie entre une valeur minimale et une valeur maximale en fonction des variations de la ventilation-minute. Cependant, c'est seulement à l'occasion d'une nouvelle intervention que le médecin peut modifier la fonction linéaire et la pente de la droite, qui restent intangibles une fois programmées.

Le document EP-0 299 208 décrit un stimulateur de ce type dans lequel la droite représentative de la fréquence de stimulation en fonction du signal reçu par le capteur d'un paramètre physiologique est définie au moyen de deux tests d'effort dans des conditions différentes d'activité du patient. Mais dans ce cas, également, la pente de la droite représentative ne peut être modifiée que par une intervention du médecin.

Le document WO-A-9 008 569 décrit un stimulateur cardiaque à fréquence asservie à un paramètre physiologique du patient selon une relation linéaire définie par un point bas et une pente programmée. Le point bas correspond à la fréquence de base et à une valeur de seuil à partir duquel le signal du capteur peut être pris en compte pour servir de paramètre d'asservissement.

Un but de la présente invention est de proposer un procédé de commande d'un stimulateur cardiaque dans lequel la fréquence d'asservissement cardiaque soit une fonction linéaire de la ventilation-minute et dans lequel la pente de la droite représentative de cette fonction linéaire soit automatiquement modifiable en cours de fonctionnement.

La présente invention a pour objet un procédé de commande d'un stimulateur cardiaque à fréquence de stimulation asservie (FC), par l'intermédiaire d'un capteur, à un paramètre physiologique X du patient, selon une relation d'asservissement, dont les coefficients sont, à tout instant et de manière automatique, contrôlés c'est-à-dire calculés et éventuellement modifiés, deux valeurs de la fréquence (FC bas et FC max) étant fixes, définies et programmées, la relation d'asservissement étant linéaire et définie par la connaissance d'un couple de valeurs (FC bas, X bas), caractérisé en ce que la relation d'asservissement est définie par un deuxième couple de valeur (FCmax, Xmax), et en ce que les valeurs Xbas et Xmax sont calculées à tout instant directement à partir de la valeur du paramètre physiologique X délivrée par le capteur et correspondant respectivement à l'activité minimale (Xbas) et à l'activité maximale (Xmax) du patient.

Selon d'autres caractéristiques de l'invention :
- le paramètre X est la ventilation-minute VE et l'on initialise l'asservissement en calculant VE bas à la valeur moyenne prise sur 32 cycles de respiration du patient au repos, et en fixant VE max = 6. VE bas ;
- la ventilation-minute VE est calculée à chaque cycle respiratoire, et une valeur moyenne VE8 des 8 derniers VE est calculée tous les 8 cycles respiratoires et comparée à la valeur de VE max en cours d'utilisation, puis, si VE8 est supérieure à VE max, la valeur de VE max sera modifiée et prise égale à la plus petite des valeurs VE8 ou Z.VE max, avec Z compris entre 1,03 et 1,12, et de préférence égal à 1,06, tandis que si VE8 est inférieure à VE max on conserve la valeur de VE max ;
- l'on baisse la valeur de VE max de l'ordre de 3% à 6%, et de préférence de 3%, si celle-ci n'a pas été modifiée pendant une durée de l'ordre de 12 ou 24 heures, et de préférence de 24 heures ;
- la valeur de VE max est comprise entre deux limites : d'une part elle est inférieure à la valeur maximale théorique de la ventilation MVV = 16.VE bas ; d'autre part, elle est supérieure à une valeur minimale programmée en fonction du patient, et égale à Y.VE bas, avec Y = 2, 3, 4 ou 5 ;
- la relation d'asservissement est recalculée après chaque modification de VE max, dès que la fréquence cardiaque du patient est inférieure à une fréquence de seuil définie proche de la fréquence basse ;
- afin de contrôler la valeur de VE bas, on calcule tous les 32 cycles respiratoires la valeur moyenne VE64 de VE au cours des 64 cycles précédents, on compare cette valeur à (VE bas ± x%), avec x compris entre 3 et 9 et de préférence égal à 6, puis, soit on prend pour nouvelle valeur (VE bas-x%) si VE64 est inférieure à (VE bas-x%), soit on incrémente un compteur lorsque VE 64 est supérieure à (VE bas + x%). Dès que ce compteur a atteint une valeur comprise entre 4 et 12, préférentiellement 8, VE bas prend la nouvelle valeur (VE bas + x%) ;

- une valeur limite VE bas/max est calculée comme étant (VE bas/moy + y%), avec VE bas/moy égale à la valeur moyenne de 256 valeurs de VE bas relevées tous les 96 cycles respiratoires (soit un VE bas moyen tous les 24576 cycles respiratoires), et y compris entre 10 et 30, et de préférence égal à 20.

D'autres caractéristiques ressortent de la description qui suit faite avec référence au dessin annexé sur lequel on peut voir :

Fig 1 une courbe de calibration représentative de la fonction linéaire reliant la ventilation-minute VE et la fréquence d'asservissement cardiaque FC ;

Fig 2 un tracé représentatif de la calibration du point haut durant un effort ;

Fig 3 une représentation des variations de la pente après recalibration du point haut ;

Fig 4 une représentation de différentes pentes d'asservissement en calibration automatique ;

Fig 5 un tracé représentant la ventilation-minute par rapport au point bas ;

Fig 6 un tracé montrant la descente du point bas ;

Fig 7 un tracé montrant la montée du point bas après 8 dépassements du seuil ;

Fig 8 un tracé montrant la limitation de la montée du point bas à la valeur VE bas/max ;

Fig 9 une représentation de la fréquence d'asservissement cardiaque en fonction de la ventilation-minute selon deux droites de pentes différentes ;

Fig 10 un schéma logique de l'initialisation de l'asservissement ;

Fig 11 un schéma logique du fonctionnement normal ;

Fig 12 un schéma logique du calcul du point haut ;

Fig 13 un schéma logique de limitation de VE max ;

Fig 14 un schéma logique de calcul du point bas ;

Fig 15 un schéma logique de calcul de VE bas/moy.

La présente invention concerne un stimulateur cardiaque prenant en compte un paramètre physiologique qui doit être fiable dans sa capacité à rendre compte de l'activité du patient. A titre d'exemple, et pour les besoins de la description, on a pris pour paramètre la ventilation-minute (VE), c'est-à-dire le volume d'air expiré par le patient en une minute.

La ventilation-minute est une donnée physiologique, variant linéairement avec la consommation en oxygène et donc avec la fréquence cardiaque (FC).

La fréquence cardiaque (FC) calculée par le stimulateur cardiaque est donc asservie à la ventilation-minute (VE) par une relation linéaire :

$$FC = (a \cdot VE) + b.$$

Lors de la mise en route du stimulateur cardiaque, il est nécessaire de fournir des valeurs aux coefficients a et b.

De manière usuelle, les valeurs de ces coefficients sont définies lors de l'implantation du stimulateur, et basées sur des tests d'effort menés en hôpital et ne sont pas modifiées ultérieurement, sauf à l'occasion d'un nouvel examen.

Une telle méthode d'asservissement de la fréquence cardiaque ne permet pas de prendre en compte les variations de l'activité du patient, comme le sommeil, la reprise d'une activité, la pratique d'un sport, et elle est basée sur une relation dans laquelle la ventilation-minute est souvent définie à partir de mesures prises lors de tests peu représentatifs de la vie normale.

C'est pourquoi la présente invention tend à proposer un procédé de commande d'un stimulateur cardiaque capable de contrôler de manière automatique, les coefficients de la relation d'asservissement à tout instant.

Aucun paramètre n'est programmé à l'avance en dehors de la fréquence cardiaque de base FC bas et de la fréquence cardiaque maximale FC max ; tous sont en permanence réévalués et modifiés afin que la droite représentative de la relation entre la ventilation-minute VE et la fréquence de stimulation cardiaque FC soit toujours la plus proche possible des besoins du patient.

Afin de définir ladite droite représentative, on détermine deux points de celle-ci, à savoir un point bas correspondant à l'activité de base du patient, et faisant correspondre la fréquence cardiaque de base programmée à une ventilation-minute basse dite de repos, et un point haut correspondant à l'activité maximale du patient, et faisant correspondre la fréquence cardiaque maximale programmée avec la ventilation-minute maximale du patient.

Les valeurs basse et haute de la fréquence d'asservissement cardiaque sont rapidement définies, car elles correspondent respectivement aux fréquences au dessous et au dessus desquelles on ne doit pas stimuler le coeur du patient. Ces valeurs sont déterminées par le médecin pour chaque patient.

Il reste alors à définir les valeurs basse et haute de la ventilation-minute. Ces valeurs seront tout d'abord définies lors d'une initialisation, à la mise en fonctionnement du stimulateur, puis seront, selon l'invention, automatiquement modifiées au cours du temps.

Pour la mise en oeuvre de l'invention, un capteur destiné à donner à tout moment, la valeur de la ventilation-minute, est implanté, et relié au stimulateur.

Dès que ce capteur est opérationnel, c'est-à-dire après implantation du stimulateur et programmation de la mise en fonctionnement du capteur, l'initialisation est déclenchée.

Afin de définir la valeur basse VE bas, le système mesure la valeur de VE durant 32 cycles respiratoires et calcule la moyenne de ces valeurs, tandis que le patient reste au repos.

La valeur basse VE bas de la ventilation-minute, sera égale à la valeur moyenne ainsi calculée.

La valeur haute VE max de la ventilation-minute, sera définie en fixant VE max à 6 fois VE bas. Une telle valeur est définie en tenant compte du fait que la valeur maximale théorique de la ventilation, qui n'est en pratique jamais atteinte par une personne dans la vie courante, est comprise entre 10 et 12 fois celle de la ventilation-minute de repos. La ventilation-minute maximale atteinte dans la vie courante, est généralement comprise entre 25 et 50% de cette valeur maximale théorique, c'est pourquoi, on fixe a priori que VE max est égale à 6 fois VE bas.

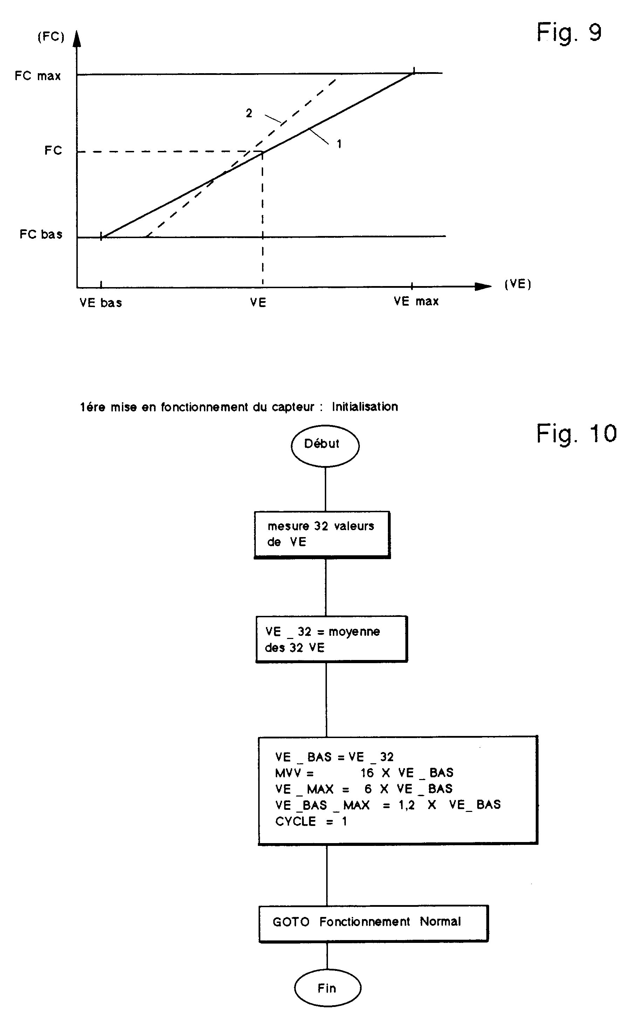

Le système connaissant FC bas, FC max, VE bas et VE max définit alors une droite représentant la relation linéaire entre la fréquence cardiaque et la ventilation-minute. Cette droite est représentée en 1 sur la figure 9, c'est la droite d'asservissement d'initialisation. Au cours du fonctionnement, pour chaque valeur de VE calculée, le stimulateur calcule la valeur correspondante de la fréquence de stimulation qu'il applique au coeur du patient.

Selon l'invention, le stimulateur va, à tout moment au cours de son fonctionnement, recalculer et modifier si nécessaire les valeurs de VE bas et VE max afin d'être toujours le plus proche possible de l'état du patient et de son besoin métabolique. Il est en effet nécessaire d'adapter la fréquence cardiaque à l'activité du patient. Si celui-ci est en phase d'effort, le rythme cardiaque doit s'accélérer afin d'assurer une oxygénation suffisante du sang; tandis que s'il est en tra in d'arrêter cet effort, il faut peu à peu diminuer la fréquence cardiaque alors que la ventilation-minute est encore à un haut niveau mais décroissante.

La description qui suit est d'abord relative à la calibration automatique de VE max tendant à rapprocher VE max de la valeur réelle de la ventilation-minute maximale du patient.

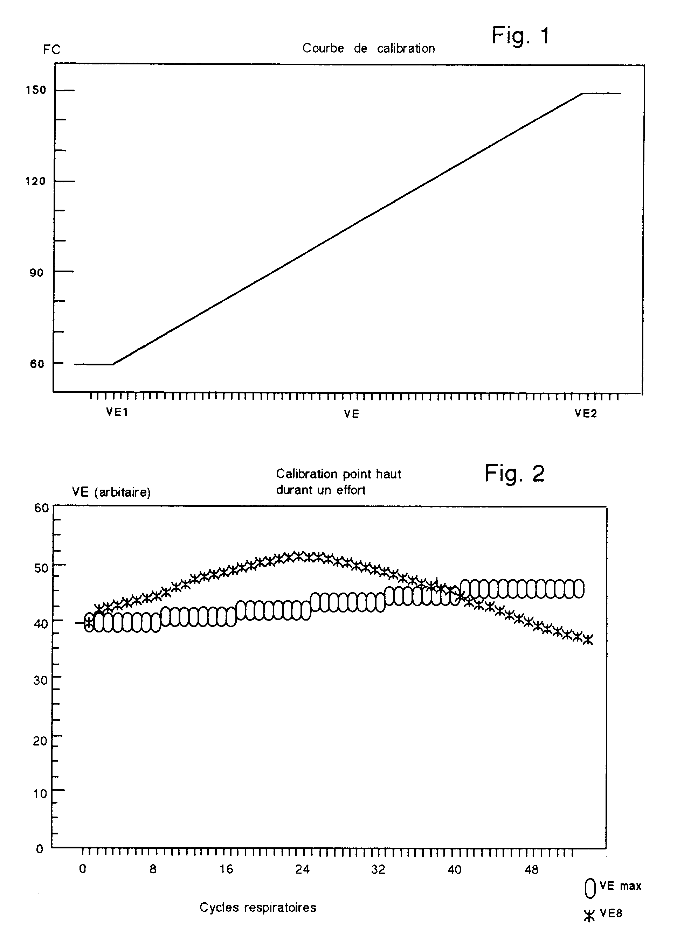

Sur la fig 1, la courbe de calibration est une relation linéaire reliant la ventilation-minute mesurée sur 4 cycles respiratoires et la fréquence cardiaque d'asservissement. Pour une ventilation-minute inférieure au point bas (VE1) la stimulation a lieu à la fréquence de base. Pour une ventilation-minute supérieure au point haut (VE2), la stimulation a lieu à la fréquence maximale. Entre les deux points est appliquée une interpolation linéaire.

La ventilation-minute VE du patient est mesurée à chaque cycle respiratoire puis, une valeur moyenne sur les 8 dernières VE, soit VE8, est calculée tous les 8 cycles respiratoires, et est comparée à la valeur actuelle de VE max.

Si la valeur moyenne VE8 calculée, est supérieure à VE max, alors il faut augmenter VE max. A cet effet, on calcule une valeur égale à 1,03 à 1,12, et de préférence 1,06. VE max, et on prend comme nouvelle valeur pour VE max la plus petite des valeurs VE8 ou 1,06 VE max.

Si par contre VE8 calculée est inférieure à VE max, la valeur de VE max n'est pas modifiée.

Sur la fig 2, tous les 8 cycles, la ventilation-minute moyennée sur 8 cycles VE8 est comparée au point haut VE max. Si VE8 est supérieure à VE max, le point haut est augmenté de 6%. Durant un effort important, VE max peut être augmentée plusieurs fois consécutivement de 6%.

Lorsqu'au cours de 12 ou 24 heures, et de préférence de 24 heures, il n'a pas été nécessaire de modifier VE max, alors on baisse sa valeur de 3% ou 6%, de préférence 3%.

Lors des modifications de VE max qui viennent d'être décrites, on vérifie toujours que sa valeur reste dans une fourchette définie à l'avance. Cette fourchette a pour limite supérieure la valeur maximale théorique de la ventilation MVV que l'on fixe à 16 fois la valeur de VE bas.

La limite inférieure de VE max est fixée par le médecin à Y. VE bas (Y pouvant prendre les valeurs 2, 3, 4, 5 fixées par le médecin), selon l'état physique du patient afin que VE max ne descende pas trop bas lors d'un arrêt prolongé d'activité.

Ainsi, si VE max devient supérieure à MVV on fixera sa valeur à celle de MVV tandis que si elle devient inférieure au seuil défini, elle sera fixée à la valeur de ce seuil.

Lorsque VE max est modifiée, la relation liant la fréquence cardiaque FC à la ventilation-minute VE n'est pas immédiatement recalculée.

En effet, on vérifie tout d'abord que le patient n'est pas en cours d'effort, ce qui pourrait faire encore augmenter la respiration et donc VE max.

La relation d'asservissement entre FC et VE sera recalculée lorsque le patient aura cessé tout effort. Le retour au repos est défini lorsque la fréquence cardiaque est redevenue inférieure à un seuil de fréquence. La période de seuil est définie comme égale à la période de base diminuée de 6%. La fréquence seuil est égale à 60.000/période de seuil lorsque la période est exprimée en millisecondes.

La relation d'asservissement sera ainsi calculée lorsque VE max est modifiée et le retour au repos constaté.

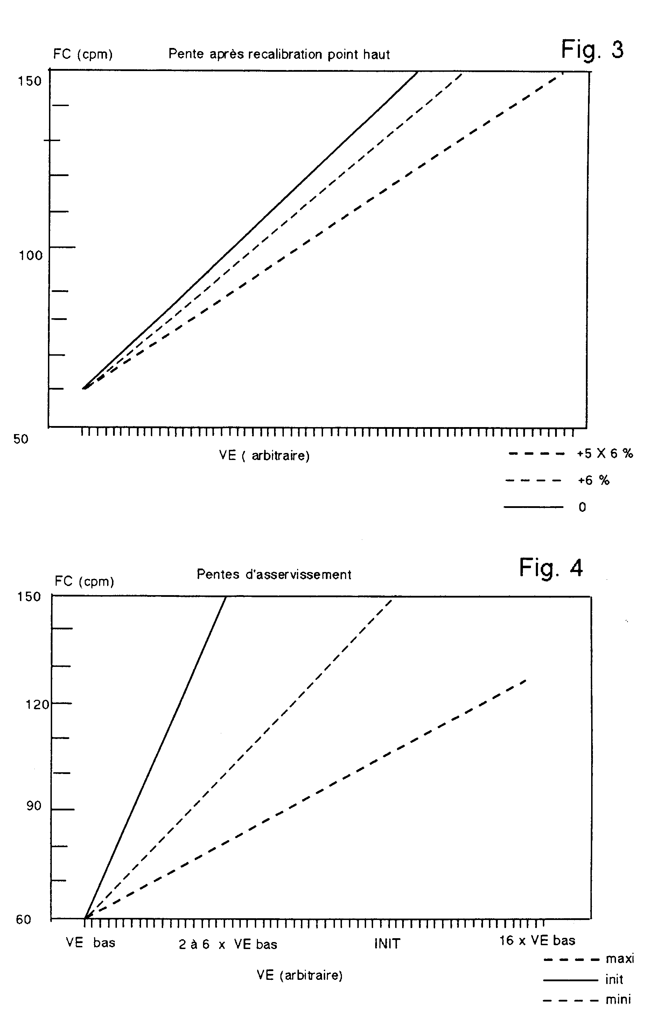

Sur la fig 3, dès que le système revient à son état de repos, une nouvelle pente de calibration est évaluée si le point haut a été modifié. Ce dernier peut avoir augmenté de 6% ou de x fois 6% (ici 5 fois).

Et sur la fig 4, en calibration automatique, le point haut peut évoluer librement suivant l'activité du patient entre 16 fois le point bas et une valeur programmée de 2 à 6 fois le point bas.

La calibration de VE bas va maintenant être décrite. La ventilation-minute de repos varie en fait très peu. Ses modifications sont soit de courte durée, soit dues à une évolution à long terme engendrée par une maladie ou un vieillissement du patient, soit dues à des modifications électriques au niveau de la sonde cardiaque, en raison de la fibrose. Le système doit également ne pas confondre un effort continu et prolongé avec une augmentation de la ventilation-minute de repos.

A cet effet, tous les 32 cycles respiratoires on calcule la valeur VE 64, moyenne de VE au cours des 64 cycles précédents.

Cette valeur est comparée à (VE bas ± 6%) afin de ne pas modifier VE bas pour des variations minimes.

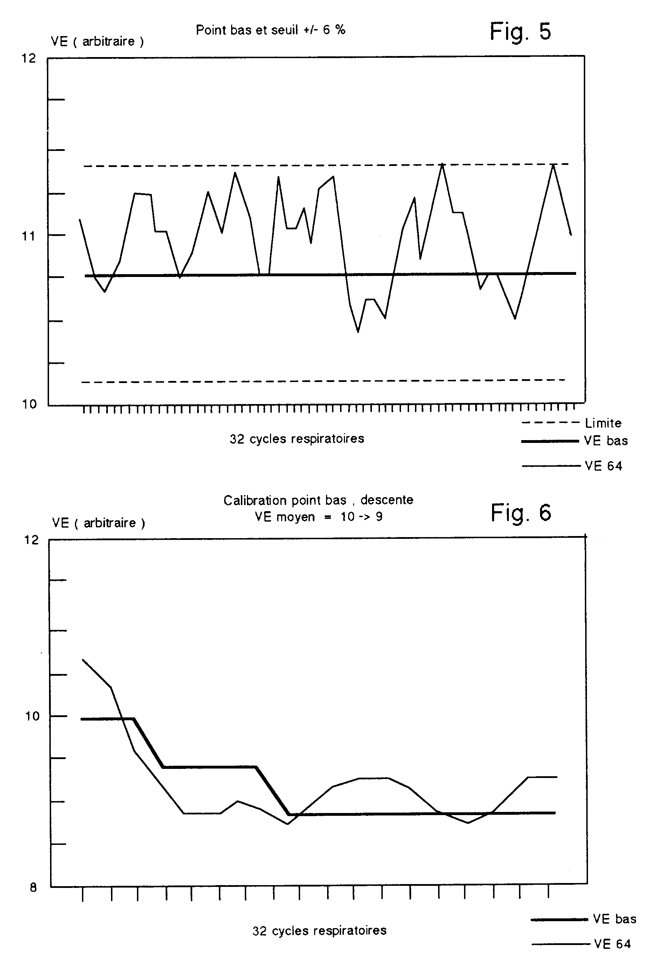

Si VE64 est compris dans la fourchette (VE bas ±6%) on ne modifie pas VE bas (Fig5).

Si VE64 est inférieure à (VE bas -6%), on considère que la ventilation de repos a baissé et on prend pour nouvelle valeur de VE bas la valeur dudit seuil inférieur, c'est-à-dire 6% en dessous du VE bas précédent.

Sur la fig 6, dès que VE64 descend sous la limite du point bas, ce dernier est diminué de 6%.

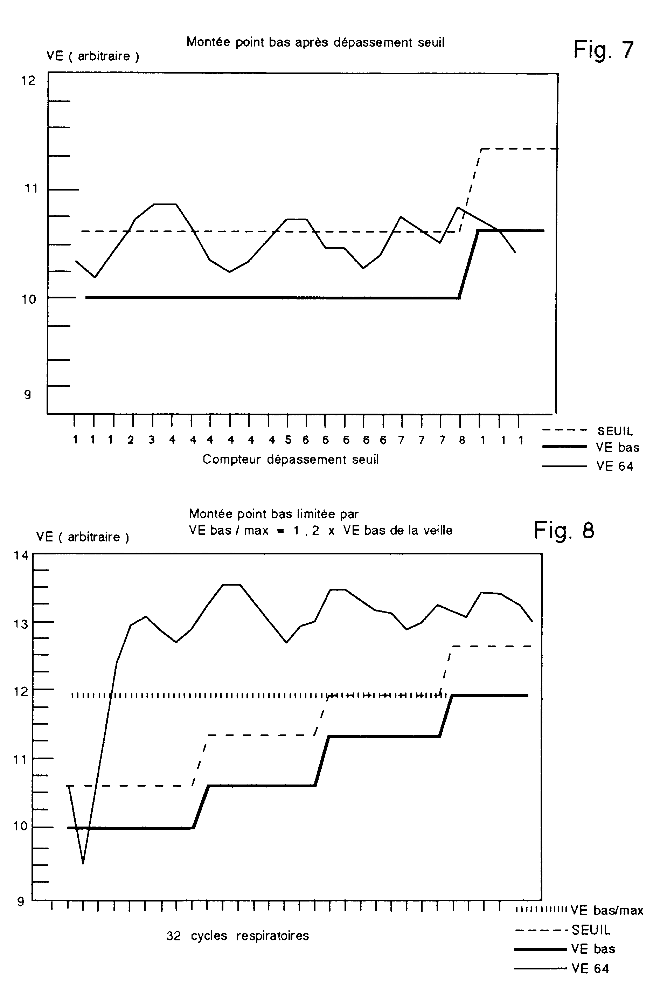

Si par contre, VE64 est supérieure au seuil supérieur de VE bas, (VE bas +6%), on incrémente un compteur. Dès que le compteur atteint 8, c'est-à-dire que VE 64 a été huit fois supérieure à (VE bas +6%), alors on prend pour nouvelle valeur le seuil supérieur soit 6% au dessus de la VE bas précédente (Fig 7). Le compteur est remis à zéro si VE bas est réduite ou augmentée de 6% par application de l'une des procédures précédentes (voir Fig. 6).

Au cours d'une telle modification, on vérifie que VE bas reste toujours inférieure à une limite VE bas/max, calculée chaque jour.

Tous les 96 cycles respiratoires, la valeur de VE bas est mémorisée, puis on calcule la valeur moyenne VE bas/moy de 256 telles valeurs. Cette valeur VE bas/moy est donc calculée tous les 24 576 cycles respiratoires, ce qui correspond à un peu plus d'une journée.

La valeur VE bas/max est alors définie comme étant y% plus forte que VE bas/moy. On prendra par exemple y = 20% et VE bas/max = 1,2.VE bas/moy. VE bas/max ainsi calculée est la valeur maximale que peut prendre VE bas quelle que soit la valeur de VE64 (Fig 8). On vérifie tous les 32 cycles que VE bas n'a pas dépassé cette valeur.

Lorsque VE bas est modifiée, on recalcule VE max et la valeur maximale théorique de la ventilation MVV afin de vérifier que VE max est toujours dans les limites imposées, et la modifier si nécessaire.

Celà étant vérifié, la relation liant la fréquence cardiaque FC à la ventilation minute VE est recalculée afin que le coeur soit stimulé à la fréquence appropriée.

La droite 2 de la figure 9 représente un exemple de relation d'asservissement entre la fréquence cardiaque FC et la ventilation-minute VE après modification des valeurs de VE bas et de VE max.

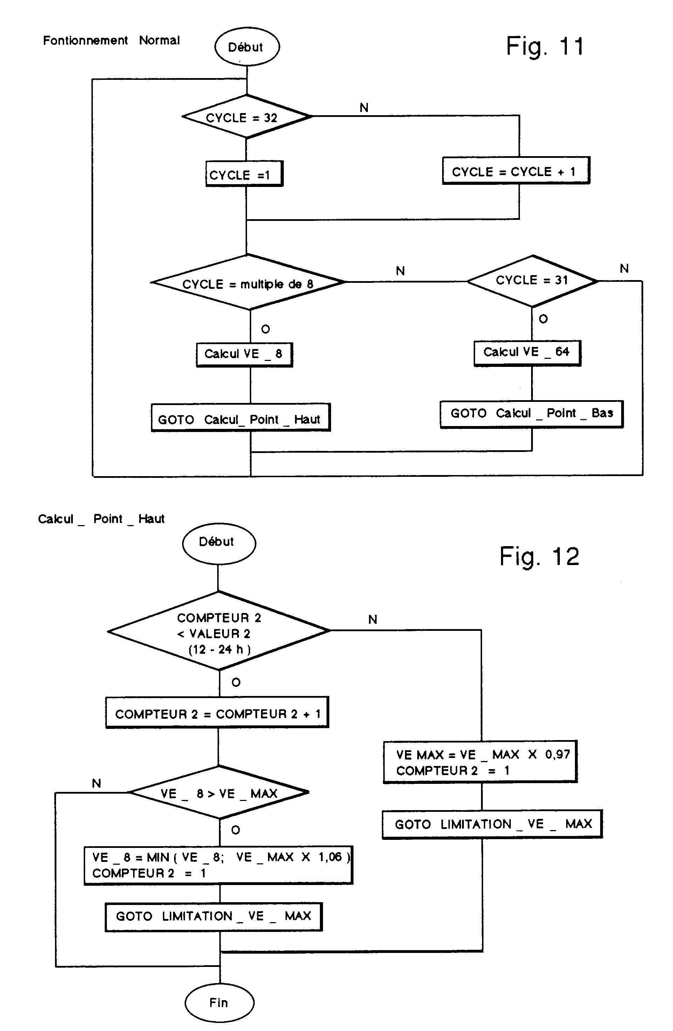

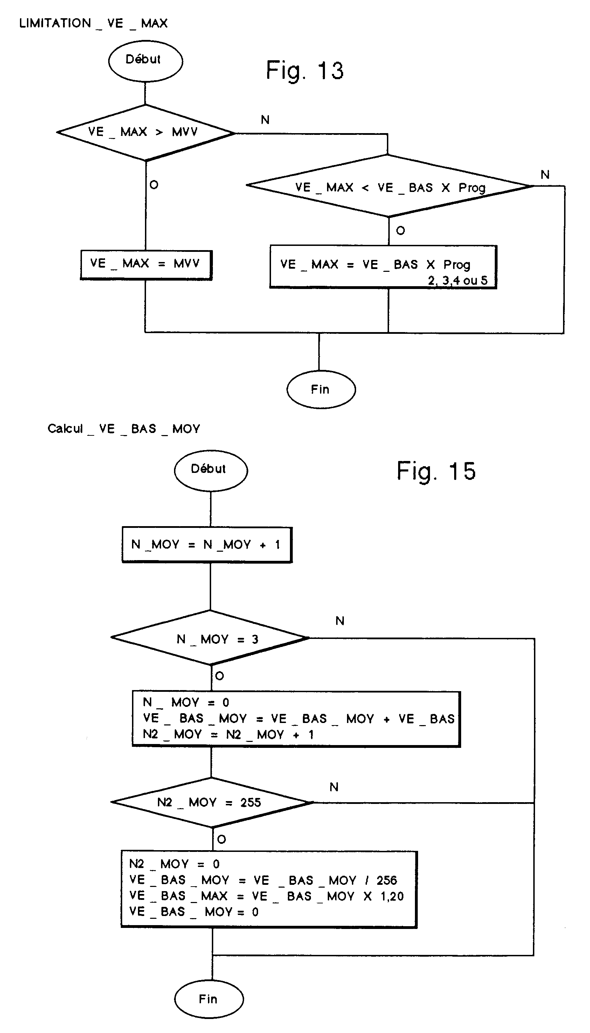

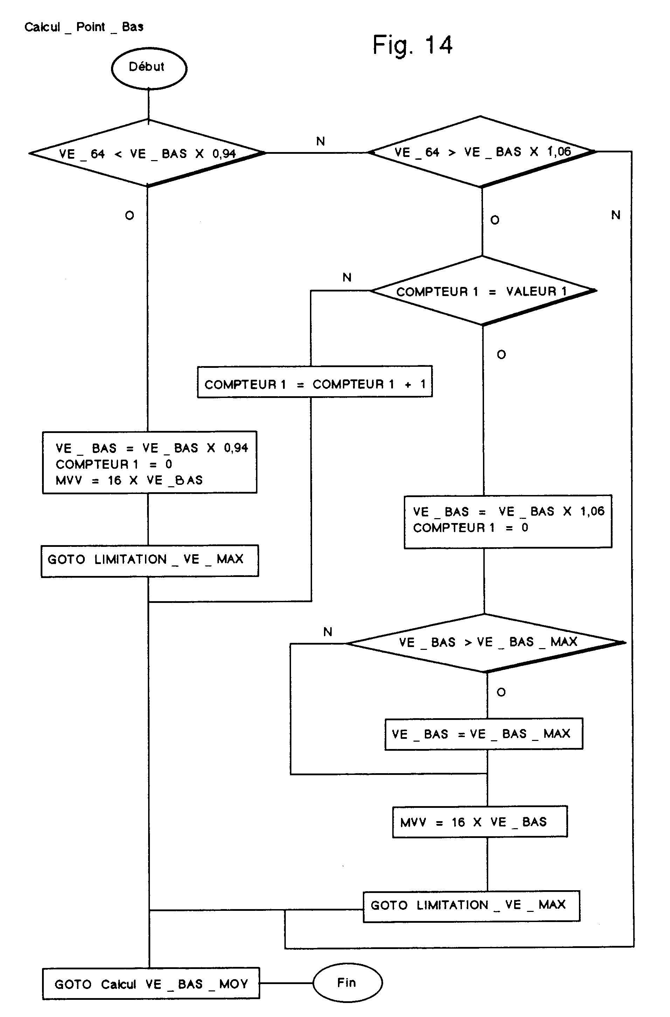

Les figures 10 à 15 illustrent les schémas logiques des différentes opérations liées au fonctionnement du stimulateur cardiaque, et décrites en relation avec les figures 1 à 8.

Le fonctionnement du stimulateur a été décrit en fonction d'un paramètre physiologique qui est la ventilation-minute du patient. Ce fonctionnement s'analyse de la même manière et fournit les mêmes résultats avec tout paramètre physiologique représentatif de l'activité du patient, tel que, par exemple, la fréquence respiratoire ou la période de pré-éjection.

**Revendications**

1. Procédé de commande d'un stimulateur cardiaque à fréquence de stimulation asservie (FC), par l'intermédiaire d'un capteur, à un paramètre physiologique (X) du patient, selon une relation d'asservissement, dont les coefficients sont, à tout instant et de manière automatique, contrôlés c'est-à-dire calculés et éventuellement modifiés, deux valeurs de la fréquence (FC bas et FC max) étant fixes, définies et programmées, la relation d'asservissement étant linéaire et définie par la connaissance d'un couple de valeurs (FC bas, X bas), caractérisé en ce que la relation d'asservissement est définie par un deuxième couple de valeur (FCmax, Xmax), et en ce que les valeurs Xbas et Xmax sont calculées à tout instant directement à partir de la valeur du paramètre physiologique (X) délivrée par le capteur et correspondant respectivement à l'activité minimale (Xbas) et à l'activité maximale (Xmax) du patient.

2. Procédé selon la revendication 1, caractérisé en ce que le paramètre X est la ventilation-minute VE et l'on initialise l'asservissement en calculant VE bas à la valeur moyenne prise sur 32 cycles de respiration du patient au repos, et en fixant VE max = 6. VE bas.

3. Procédé selon la revendication 2 caractérisé en ce que la ventilation-minute VE est calculée à chaque cycle respiratoire, et en ce qu'une valeur moyenne VE8 des 8 derniers VE est calculée tous les 8 cycles respiratoires et comparée à la valeur de VE max en cours d'utilisation, puis, si VE8 est supérieure à VE max, la valeur de VE max sera modifiée et prise égale à la plus petite des valeurs VE8 ou Z.VE max, avec Z compris entre 1,03 et 1,12, et de préférence égal à 1,06, tandis que si VE8 est inférieure à VE max on conserve la valeur de VE max.

4. Procédé selon la revendication 3, caractérisé en ce que l'on baisse la valeur de VE max de l'ordre de 3% à 6%, et de préférence de 3%, si celle-ci n'a pas été modifiée pendant une durée de l'ordre de 12 ou 24 heures, et de préférence de 24 heures.

5. Procédé selon l'une des revendications 3 et 4, caractérisé en ce que la valeur de VE max est comprise entre deux limites : d'une part, elle est inférieure à la valeur maximale théorique de la ventilation MVV = 16.VE bas; d'autre part, elle est supérieure à une valeur minimale programmée en fonction du patient et égale à Y.VE bas, avec Y = 2, 3, 4 ou 5.

6. Procédé selon l'une des revendications 2 à 5 caractérisé en ce que la relation d'asservissement est recalculée après chaque modification de VE max, dès que la fréquence cardiaque du patient est inférieure à une fréquence de seuil définie proche de la fréquence basse.

7. Procédé selon l'une des revendications 2 à 6, caractérisé en ce que afin de contrôler la valeur de VE bas, on calcule tous les 32 cycles respiratoires la valeur moyenne VE 64 de VE au cours des 64 cycles précédents, on compare cette valeur à (VE bas ± x%), avec x compris entre 3 et 9 et de préférence égal à 6, puis, soit on prend pour nouvelle valeur (VE bas-x%) si VE 64 est inférieure à (VE bas-x%), soit on incrémente un compteur lorsque VE64 est supérieure à (VE bas + x%), et dès que ce compteur a atteint une valeur comprise entre 4 et 12, et de préférence 8, on prend pour nouvelle valeur de VE bas la valeur (VE bas + x%).

8. Procédé selon l'une des revendications 2 à 7, caractérisé en ce qu'une valeur limite VE bas/max est calculée comme étant (VE bas/moy + y%), avec VE bas/moy égale à la valeur moyenne de 256 valeurs de VE bas relevées tous les 96 cycles respiratoires, et y compris entre 10 et 30 et de préférence égal à 20.

**Claims**

1. A method for controlling a cardiac pacemaker with pacing frequency automatically controlled (FC), by means of a sensor, by a physiological parameter (X) of the patient according to a control relationship, the coefficients of which are, at any moment and automatically, controlled, that is to say calculated and possibly modified, two values of the frequency (FC bas and FC max) being fixed, defined and programmed, the control relationship being linear and defined by the knowledge of a pair of values (FC bas, X bas), characterised in that the control relationship is defined by a second pair of values (FCmax, Xmax), and in that the values Xbas and Xmax are calculated at any moment directly from the value of the physiological parameter (X) provided by the sensor and corresponding respectively to the minimum activity (Xbas) and to the maximum activity (Xmax) of the patient.

2. A method according to Claim 1, characterised in that the parameter X is the minute ventilation VE and the automatic control is initialised by calculating VE bas at the mean value taken over 32 respiration cycles of the patient at rest, and by fixing VE max = 6. VE bas.

3. A method according to Claim 2, characterised in that the ventilation-minute VE is calculated at each respiratory cycle, and in that an mean value VE8 of the last 8 VE is calculated every 8 respiratory cycles and compared with the value of VE max currently being used, then, if VE8 is greater than VE max, the value of VE max will be modified and taken to be equal to the smaller of the values VE8 or Z.VE max, with Z being between 1.03 and 1.12, and preferably equal to 1.06, whereas if VE8 is less than VE max the value of VE max is retained.

4. A method according to Claim 3, characterised in that the value of VE max is lowered by the order of 3% to 6%, and preferably by 3%, if the latter has not been modified during a time of the order of 12 or 24 hours, and preferably 24 hours.

5. A method according to one of Claims 3 and 4, characterised in that the value of VE max lies between two limits: firstly, it is less than the theoretical maximum value of ventilation MVV = 16.VE bas; secondly, it is greater than a minimum value programmed as a function of the patient and equal to Y.VE bas, with Y = 2, 3, 4 or 5.

6. A method according to one of Claims 2 to 5, characterised in that the control relationship is recalculated after each modification of VE max, as soon as the heart rate of the patient is lower than a defined threshold rate close to the low rate.

7. A method according to one of Claims 2 to 6, characterised in that in order to control the value of VE bas, the mean value VE 64 of VE during the preceding 64 cycles is calculated every 32 respiratory cycles, this value is compared to (VE bas ± x%), x being between 3 and 9 and preferably equal to 6, then either (VE bas-x%) is taken for the new value if VE 64 is less than (VE bas-x%), or a counter is incremented when VE64 is greater than (VE bas + x%), and as soon as this counter has reached a value of between 4 and 12, and preferably 8, the value (VE bas + x%) is taken as the new value of VE bas.

8. A method according to one of Claims 2 to 7, characterised in that a limit value VE bas/max is calculated as being (VE bas/moy + y%), with VE bas/moy being equal to the mean value of 256 values of VE bas recorded every 96 respiratory cycles, and included between 10 and 30 and preferably equal to 20.

**Patentansprüche**

1. Verfahren zur Steuerung eines Herzschrittmachers mit mittels eines Meßwertaufnehmers von einem physiologischen Parameter (X) des Patienten gemäß einer Steuerungsbeziehung gesteuerter Stimulationsfrequenz (FC), deren Koeffizienten zu jedem Zeitpunkt und automatisch gesteuert, d.h. berechnet und ggf. modifiziert werden, wobei zwei Werte der Frequenz (FCbas und FCmax) festliegen, definiert und programmiert sind, die Steuerungsbeziehung linear ist und durch die Kenntnis eines Wertepaares (FCbas, Xbas) definiert ist, **dadurch gekennzeichnet, daß** die Steuerungsbeziehung durch ein zweites Wertepaar (FCmax, Xmax) definiert ist, und daß die Werte Xbas und Xmax jederzeit direkt ausgehend von dem Wert des mittels des Meßwertaufnehmers gelieferten physiologischen Parameters (X) berechnet werden und jeweils der minimalen Aktivität (Xbas) und der maximalen Aktivität (Xmax) des Patienten entsprechen.

2. Verfahren gemäß Patentanspruch 1, **dadurch gekennzeichnet, daß** der Parameter X die Minuten-Ventilation VE ist und die Steuerung initialisiert wird, indem VEbas als der über 32 Atmungszyklen des Patienten im Ruhezustand ermittelte Mittelwert berechnet wird, und indem VEmax = 6. VEbas festgelegt wird.

3. Verfahren gemäß Patentanspruch 2, **dadurch gekennzeichnet, daß** die Minuten-Ventilation VE bei jedem Atmungszyklus berechnet wird, und dadurch, daß ein Mittelwert VE8 der 8 letzten VE alle 8 Atmungszyklen berechnet und während der Verwendung mit dem Wert VEmax verglichen wird, weiterhin daß, wenn VE8 größer als VEmax ist, der Wert von VEmax modifiziert wird und als gleich dem kleinsten der Werte VE8 oder Z.VEmax angenommen wird, wobei Z zwischen 1,03 und 1,12 liegt und vorzugsweise 1,06 beträgt, während, wenn VE8 kleiner als VEmax ist, der Wert von VEmax beibehalten wird.

4. Verfahren gemäß Patentanspruch 3, **dadurch gekennzeichnet, daß** der Wert von VEmax in der Größenordnung von 3% bis 6% und vorzugsweise um 3% verringert wird, wenn dieser während einer Dauer in der Größenordnung von 12 oder 24 Stunden, vorzugsweise 24 Stunden, nicht modifiziert wurde.

5. Verfahren gemäß einem der Patentansprüche 3 und 4, **dadurch gekennzeichnet, daß** der Wert von VEmax zwischen zwei Grenzen liegt: einerseits ist er kleiner als der theoretische maximale Wert der Ventilation MVV = 16. VEbas; andererseits ist er größer als ein programmierter minimaler Wert in Abhängigkeit des Patienten und gleich Y.VEbas, wwobei Y = 2, 3, 4 oder 5 ist.

6. Verfahren gemäß einem der Patentansprüche 2 bis 5, **dadurch gekennzeichnet, daß** die Steuerungsbeziehung nach jeder Modifikation von VEmax neu berechnet wird, sobald die Herzfrequenz des Patienten kleiner als eine nahe der Niederfrequenz definierte Schwellenfrequenz ist.

7. Verfahren gemäß einem der Patentansprüche 2 bis 6,
**dadurch gekennzeichnet, daß**
zum Steuern des Wertes von VEbas alle 32 Atmungszyklen der Mittelwert VE64 von VE im Verlauf der 64 vorhergehenden Zyklen berechnet wird, dieser Wert mit (VEbas + - x%) verglichen wird, wobei x zwischen 3 und 9 liegt und vorzugsweise 6 ist, dann, entweder (VEbas - x%) als neuer Wert angenommen wird, wenn VE64 kleiner als (VEbas - x%) ist, oder ein Zähler inkrementiert wird, wenn VE64 größer ist als (VEbas + x%), und sobald der Zähler einen zwischen 4 und 12 liegenden und vorzugsweise 8 betragenden Wert erreicht hat, der Wert (VEbas + x%) als neuer Wert von VEbas angenommen wird.

8. Verfahren gemäß einem der Patentansprüche 2 bis 7,
**dadurch gekennzeichnet, daß**
ein Grenzwert VEbas/max als (VEbas/moy + y%) berechnet wird, wobei VEbas/moy gleich einem Mittelwert von 256 Werten von VEbas ist, die alle 96 Atmungszyklen abgelesen werden und y zwischen 10 und 30 liegt und vorzugsweise 20 ist.

## Fig. 1

FC

Courbe de calibration

## Fig. 2

VE (arbitaire)

Calibration point haut
durant un effort

Cycles respiratoires

◯ VE max

✳ VE8

Fig. 3

Pente après recalibration point haut

FC (cpm)

VE ( arbitraire)

- - - - +5 X 6 %
- - - - +6 %
——— 0

Fig. 4

Pentes d'asservissement

FC (cpm)

VE bas    2 à 6 x VE bas    INIT    16 x VE bas

VE (arbitraire)

- - - - maxi
——— init
- - - - mini

EP 0 493 222 B1

Fig. 5

Point bas et seuil +/- 6 %

VE ( arbitraire )

32 cycles respiratoires

Limite
VE bas
VE 64

Fig. 6

Calibration point bas , descente
VE moyen = 10 -> 9

VE ( arbitraire )

32 cycles respiratoires

VE bas
VE 64

11

Montée point bas après dépassement seuil

Fig. 7

VE ( arbitraire )

Compteur dépassement seuil

- - - - SEUIL
—— VE bas
—— VE 64

Montée point bas limitée par
VE bas / max = 1 , 2 x VE bas de la veille

Fig. 8

VE ( arbitraire )

32 cycles respiratoires

IIIIIIIIIIII VE bas/max
- - - - - SEUIL
—— VE bas
—— VE 64

Fig. 9

Fig. 10

1ére mise en fonctionnement du capteur : Initialisation

Fontionnement Normal

Fig. 11

Début

CYCLE = 32 ──N──

CYCLE = 1

CYCLE = CYCLE + 1

CYCLE = multiple de 8 ──N── CYCLE = 31 ──N──

O

O

Calcul VE _ 8

Calcul VE _ 64

GOTO Calcul _ Point _ Haut

GOTO Calcul _ Point _ Bas

Calcul _ Point _ Haut

Fig. 12

Début

COMPTEUR 2
< VALEUR 2
( 12 - 24 h ) ──N──

O

COMPTEUR 2 = COMPTEUR 2 + 1

N── VE _ 8 > VE _ MAX

O

VE MAX = VE _ MAX X 0,97
COMPTEUR 2 = 1

VE _ 8 = MIN ( VE _ 8; VE _ MAX X 1,06 )
COMPTEUR 2 = 1

GOTO LIMITATION _ VE _ MAX

GOTO LIMITATION _ VE _ MAX

Fin

LIMITATION _ VE _ MAX

## Fig. 13

```
        ( Début )
            │
            ▼
      ◇ VE _ MAX > MVV ◇────── N ──────┐
            │                           │
            O                           ▼
            │              ◇ VE _ MAX < VE _ BAS X Prog ◇──── N ──┐
            ▼                           │                         │
  ┌──────────────────┐                 O                         │
  │ VE _ MAX = MVV   │     ┌────────────────────────────────┐    │
  └──────────────────┘     │ VE _ MAX = VE _ BAS X Prog     │    │
            │              │                  2, 3,4 ou 5    │    │
            │              └────────────────────────────────┘    │
            └───────────────────┬──────────────────┘─────────────┘
                                ▼
                            ( Fin )
```

Calcul _ VE _ BAS _ MOY

## Fig. 15

```
              ( Début )
                  │
                  ▼
        ┌──────────────────────┐
        │ N _MOY = N _MOY + 1  │
        └──────────────────────┘
                  │
                  ▼
          ◇ N _ MOY = 3 ◇─────── N ──────────┐
                  │                           │
                  O                           │
                  ▼                           │
  ┌──────────────────────────────────────┐   │
  │ N _ MOY = 0                           │   │
  │ VE _ BAS _ MOY = VE _ BAS _ MOY + VE _ BAS │
  │ N2 _ MOY = N2 _ MOY + 1               │   │
  └──────────────────────────────────────┘   │
                  │                           │
                  ▼                           │
          ◇ N2 _ MOY = 255 ◇──── N ───────────┤
                  │                           │
                  O                           │
                  ▼                           │
  ┌──────────────────────────────────────┐   │
  │ N2 _ MOY = 0                          │   │
  │ VE _ BAS _ MOY = VE _ BAS _MOY / 256  │   │
  │ VE _ BAS _ MAX = VE _ BAS _ MOY X 1,20│   │
  │ VE _ BAS _ MOY = 0                    │   │
  └──────────────────────────────────────┘   │
                  │                           │
                  └───────────┬───────────────┘
                              ▼
                          ( Fin )
```

Calcul _ Point _ Bas

## Fig. 14

Début

VE _ 64 < VE _ BAS X 0,94 — N — VE _ 64 > VE _ BAS X 1,06

O

N

COMPTEUR 1 = VALEUR 1

O

N

COMPTEUR 1 = COMPTEUR 1 + 1

```
VE _ BAS = VE _ BAS X 0,94
COMPTEUR 1 = 0
MVV = 16 X VE _ BAS
```

```
VE _ BAS = VE _ BAS X 1,06
COMPTEUR 1 = 0
```

GOTO LIMITATION _ VE _ MAX

N — VE _ BAS > VE _ BAS _ MAX

O

VE _ BAS = VE _ BAS _ MAX

MVV = 16 X VE _ BAS

GOTO LIMITATION _ VE _ MAX

GOTO Calcul VE _ BAS _ MOY — Fin